# FASCICULE DE BREVET EUROPEEN

(11) **EP 2 911 602 B1**
(45) Date de publication et mention de la délivrance du brevet: **05.10.2016**
(21) Numéro de dépôt: 13785484.0
(22) Date de dépôt: 23.09.2013
(51) Int. Cl.: A61B 17/80

(54) **PLAQUE DE TRANSLATION DU CALCANÉUM**
FERSENBEINTRANSLATIONSPLATTE
CALCANEUM TRANSLATION PLATE

(30) Priorité: 29.10.2012 FR 1260297
(43) Date de publication de la demande: 02.09.2015
(73) Titulaire: BIOTECH ORTHO, 13300 Salon-de-Provence (FR)
(72) Inventeur: LEEMRIJSE, Thibaut, B-1200 Bruxelles (BE); MAESTRO, Michel, F-06200 Nice (FR); DEVOS, Bernhard, B-9831 Deurle (BE); RELAVE, Marc, F-42160 Andrezieu Boutheon (FR); BESSE, Jean-Luc, F-69970 Chaponnay (FR)
(74) Mandataire: Weber, Etienne Nicolas
(86) Numéro de dépôt international: PCT/FR2013/052214
(87) Numéro de publication internationale: WO 2014/068206

(56) Documents cités:
- WO-A1-2010/030960
- US-A1- 2011 009 866
- US-A1- 2012 209 334

## Description

La présente invention se rattache au domaine de la chirurgie orthopédique correctrice.

Elle concerne un implant destiné aux opérations d'ostéotomie. Plus précisément, l'invention est relative à une plaque de translation calcanéenne.

Les os du squelette des êtres humains et des vertébrés en général, sont constitués d'au moins deux zones : une zone corticale rigide et une zone interne spongieuse de consistance plus tendre qui peut être elle-même, pour certains os, traversée par un canal médullaire renfermant des tissus mous.

L'arrière pied humain comprend principalement deux os : le talus (ou astragale) en haut qui est posé sur le calcanéum en bas. Le calcanéum est un os court, pair et asymétrique, de forme allongée d'arrière vers l'avant et aplati transversalement. L'association du calcanéum et du talus forme le talon sur lequel repose l'essentiel du poids du corps. Cette partie du pied est donc soumise à de fortes pressions, ce qui la rend sujette à des malformations diverses, sources de douleurs et de difficultés et/ou incapacités à marcher pour les patients.

Il est parfois indispensable de corriger la forme du calcanéum. L'intervention consiste en une ostéotomie dudit os avec médialisation de la grosse tubérosité, c'est-à-dire la section du calcanéum en deux parties et le déplacement de l'une par rapport à l'autre. Ceci va permettre un meilleur appui de l'arrière pied et surtout de diminuer les contraintes internes sur les muscles et ligaments.

L'ostéotomie désigne la section chirurgicale d'un os long, pour en modifier son axe, sa taille ou sa forme, à des fins thérapeutiques ou plastiques, elle vise à redonner de meilleurs axes aux os longs, afin de mieux répartir les pressions sur les articulations correspondantes. Ensuite, le principe est soit de découper une tranche sur la largeur de l'os, et de refixer les morceaux restant après l'avoir retirée, soit de couper simplement en travers et d'ouvrir l'espace d'un côté, en comblant le vide créé avec un greffon osseux, selon que l'on veut fermer un angle ou l'ouvrir. L'ostéotomie peut aussi être suivie d'une translation c'est-à-dire du décalage d'une des parties de l'os issues de cette section.

Cette opération nécessite la mise en place de systèmes de soutien du calcanéum afin de maintenir la translation en place. Dans le cas du calcanéum, cette partie du pied étant soumise à de très fortes pressions, dues au poids du corps et à son rôle dans la marche, la principale difficulté est de produire des systèmes orthopédiques suffisamment stables et rigides pour rester en place et maintenir la translation du calcanéum.

Il est connu des implants calcanéen de formes diverses et variées mais la pérennisation du système, c'est-à-dire sa stabilité et sa rigidité, est souvent incertaine car l'implant prend généralement appui dans la partie spongieuse du calcanéum, malgré les traversées thalamiques localement plus dures, ce qui engendre une rotation du système ainsi qu'un glissement de ce dernier sous l'effet des forces du poids du corps et de la porosité de la partie spongieuse de l'os.

Par exemple, le document US-2011/009.866 décrit un système pour l'ostéotomie composé d'une plaque qui comprend deux extrémités alignées le long d'un axe longitudinal se raccordant par une partie médiane et par des vis. La première extrémité comprend une arête de coupe et un trou qui reçoit une vis de non-verrouillage et présente un chanfrein qui se rétrécit à partir d'une plus faible épaisseur au niveau du bord en direction du trou, la seconde extrémité possède un trou de verrouillage qui reçoit une vis de blocage, et le premier et le second trou sont alignés le long de l'axe longitudinal.

L'inconvénient de ce dispositif est principalement le manque de stabilité : en effet, la configuration de cet implant ne lui permet pas de prendre appui sur la corticale de l'extrémité proximale du calcanéum. Il n'a donc comme seul appui que l'os spongieux qui est poreux. Au vu des forces extrêmement importantes s'appliquant sur cet os, notamment lors de la marche, ce système est susceptible d'entrer en rotation ou de glisser dans la partie spongieuse de la partie proximale du calcanéum ce qui lui confère une grande instabilité et présente des risques importants pour le patient.

On connait également des implants calcanéens n'ayant pas d'effets compressifs sur les vis orthopédiques permettant leur fixation ou pour lesquels le verrouillage desdites vis se fait avec un couple métal-métal ce qui aboutit, à long terme, à une soudure à froid du couple galvanique entrainant une corrosion toxique pour le patient et rendant impossible le retrait ultérieur des vis.

De plus, ces implants présentent l'inconvénient de nécessiter une chirurgie particulièrement invasive pour leur mise en place, en obligeant le praticien à effectuer des incisions d'ouverture importantes sur toute la partie latérale du calcanéum.

Les caractéristiques du préambule de la revendication 1 sont connues du document US 2012/0209334 A1.

Un but de la présente invention est de remédier aux inconvénients précités en fournissant un implant capable de pérenniser, c'est-à-dire de stabiliser et rigidifier, le système issu de la translation du calcanéum.

Selon l'invention, ce but est atteint grâce à une plaque ou implant de translation du calcanéum, comprenant deux parties extrêmes, soit une partie proximale d'ancrage et une partie distale de fixation, orientées dans des directions opposées et disposées dans des plans parallèles espacés, lesdites parties proximale et distale se raccordant à une troisième partie intermédiaire présentant un profil général en équerre et comportant deux surfaces d'appui perpendiculaires, la partie proximale d'ancrage s'étendant à partir de l'une de ces surfaces et perpendiculairement à celle-ci, tandis que la partie distale de fixation s'étend dans le prolongement de l'autre surface d'appui.

Selon une autre disposition caractéristique, la partie proximale d'ancrage, est constituée par une lame présentant un profil s'amincissant en direction de son extrémité libre.

Un mode de mise en oeuvre avantageux consiste à doter ladite lame de crans anti-retour empêchant la rotation de l'implant après sa mise en place. De préférence, ces crans anti-retour sont présents sur les bords latéraux de la partie proximale d'ancrage.

Selon un mode de réalisation préféré, la partie intermédiaire présente un épaulement au niveau de sa surface d'appui à la partie proximal de l'implant. Apres la mise en place de la plaque, ledit épaulement vient en butée contre la mince corticale de l'extrémité proximale du calcanéum et évite ainsi à la plaque de glisser dans la partie spongieuse de l'os.

Selon un mode de réalisation caractéristique de l'invention, la partie distale de la plaque comporte au moins un trou pour le passage d'au moins une vis de fixation.

La partie intermédiaire de la plaque peut présenter une concavité qui peut accueillir un ou plusieurs trous pour le passage d'une ou plusieurs vis de fixation. De manière avantageuse, les parties distale et intermédiaire présentent chacune deux trous de passage de vis, l'axe des trous de la partie intermédiaire étant incliné par rapport à l'axe des trous de vis de la face distale. Préférentiellement, lesdits trous ont une forme conique.

Selon un mode d'exécution préféré, la partie distale de la plaque présente un bosselage sur sa face au contact de l'os.

De manière remarquable en soi, la partie intermédiaire de ladite plaque présente sur ses bords latéraux des goussets de renfort.

Les buts, caractéristiques et avantages ci-dessus, et d'autres encore, ressortiront mieux de la description détaillée qui suit et des dessins annexés dans lesquels :
La figure 1 est une vue de face de la plaque de translation calcanéenne selon l'invention.
La figure 2 est une vue arrière de la figure 1.
La figure 3 est une vue en perspective de la plaque selon l'invention.
La figure 4 est une vue de côté de la plaque de translation calcanéenne selon l'invention.
Les figures 5 et 6 illustrent, à titre d'exemples, différents modes de réalisation de la plaque selon l'invention constituant une partie d'une gamme d'implants pouvant être présentée au praticien.
La figure 7 est une coupe longitudinale, à plus grande échelle, selon la ligne 7-7 de la figure 1.

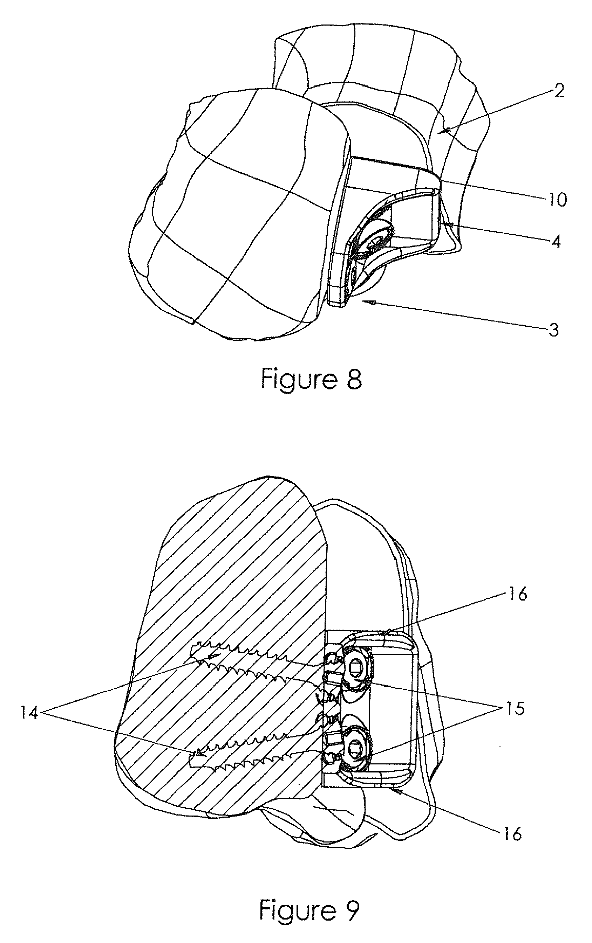

La figure 8 est une vue en perspective de l'arrière pied montrant la plaque de translation du calcanéum selon l'invention en position d'utilisation, les parties distale et proximale du calcanéum étant représentées en transparence.
La figure 9 est une vue de détail de face et à plus grande échelle, illustrant l'appui de l'épaulement de la partie intermédiaire de la plaque contre la corticale de la partie proximale du calcanéum.

On se réfère auxdits dessins pour décrire un exemple intéressant quoique nullement limitatif, de réalisation de la plaque de translation du calcanéum selon l'invention.

Le terme « distale » qualifie la partie ou extrémité d'un os qui est la plus éloignée de la racine de cet os. Le terme « proximale » qualifie la partie ou extrémité d'un os qui est la plus proche de son point de fixation. Ici, l'extrémité proximale du calcanéum est donc celle rattachée à l'astragale et au reste des os du pied, la partie ou extrémité distale du calcanéum désignant l'extrémité de l'os que l'ostéotomie sépare du reste du corps. De même, les parties proximale et distale de l'implant sont définies selon qu'elles prennent appuis sur l'extrémité proximale ou distale du calcanéum.

Selon l'invention, le but visé est atteint grâce à un implant constitué par une plaque 1 comprenant trois parties : une partie proximale d'ancrage 2, une partie distale de fixation 3 et une partie intermédiaire 4 qui les relient. Les parties distale 3 et proximale 2 étant orientées dans des directions opposées et disposées dans des plans parallèles espacés P-P, P'-P', et présentant une forme globalement rectangulaire. Le design ergonomique des plaques diminue les frottements avec la peau et la détection de l'implant une fois posé.

La première partie proximale d'ancrage 2 destinée à s'insérer dans l'os spongieux de l'extrémité proximale du calcanéum, la seconde partie est une face de fixation 3 appelée à se poser sur la surface corticale de l'extrémité distale du calcanéum pour y prendre appui, stabilisant ainsi la partie de l'os ayant été désolidarisée du reste du squelette par l'ostéotomie. Lesdites parties distale 3 et proximale 2 se raccordent à une partie intermédiaire 4 présentant un profil général en équerre et comportant deux surfaces d'appui perpendiculaires 5 et 6, la partie proximale d'ancrage s'étendant à partir de l'une de ces surfaces 5 et perpendiculairement à celle-ci, tandis que la partie distale de fixation s'étend dans le prolongement de l'autre surface d'appuis 6.

Les parties proximale 2 et distale 3 ne sont donc pas alignées mais décalées par ladite zone intermédiaire 4 qui les sépare.

Avantageusement, la partie proximale d'ancrage de la plaque selon l'invention peut être constituée par une lame 7 présentant un profil s'amincissant en direction de son extrémité libre pour former une arête de coupe.

Selon une disposition caractéristique de l'invention, la partie proximale d'ancrage 2 de l'implant, c'est-à-dire celle destinée à s'insérer dans le spongieux de l'extrémité proximale du calcanéum, présente des crans anti-retour 8, qui empêchent l'implant de se déloger et lui évitent d'entrer en rotation une fois inséré dans l'os. Ces crans anti-retour sont, par exemple, disposés sur les bords latéraux de ladite partie proximale 2.

Selon une disposition caractéristique de l'invention, la partie intermédiaire 4 de la plaque présente une concavité 9 au niveau de sa face d'appui 6 d'où s'étend la partie distale de fixation 3 de la plaque et un épaulement 10 au niveau de sa face d'appui 5 destinée à la partie proximale d'ancrage 2 de la plaque. En position d'utilisation, la partie d'ancrage 2 étant insérée dans la couche spongieuse de l'os, ledit épaulement 10 vient en appui contre la couche corticale de la partie proximale du calcanéum pour stabiliser le système et lui éviter de glisser dans cette partie spongieuse de l'os sous l'effet des forces générées par le poids du corps.

Selon un mode d'exécution préféré, la partie distale de fixation 3 de la plaque 1 présente un bosselage 11 destiné à éviter l'écrasement et donc l'endommagement du périoste. Le périoste est la membrane vascularisée qui recouvre toute la surface des os (à l'exception du cartilage articulaire) et contient les vaisseaux sanguins qui apportent les nutriments indispensables à leur réparation. Une atteinte du périoste entraine une incapacité de l'os à se régénérer, voir même sa nécrose. Ce bosselage 11 permet de sauvegarder le périoste en offrant à la plaque 1 des appuis ponctuels, et non un appui surfacique qui compresse le périoste et l'endommage, comme c'est habituellement le cas des implants calcanéens.

Selon un mode de réalisation de l'invention, la partie distale de fixation 3 de la plaque comporte au moins un trou 12 pour le passage d'au moins une vis de fixation.

Selon un exemple de réalisation, la partie intermédiaire présente une concavité 9 qui peut, de façon caractéristique, comporte un ou plusieurs trous 13 pour le passage d'une ou plusieurs vis de fixation (15). Préférentiellement, la partie distale 3 de l'implant ainsi que la partie intermédiaire 4, sont toutes deux munies de trous 12 et 13 pour le passage des vis, l'axe des trous 13 de la partie intermédiaire étant incliné par rapport à l'axe des trous 12 de la partie distale.

Par contre, la plaque de translation du calcanéum 1 selon l'invention ne comporte pas de trou dans l'extrémité de la partie proximale d'ancrage 2.

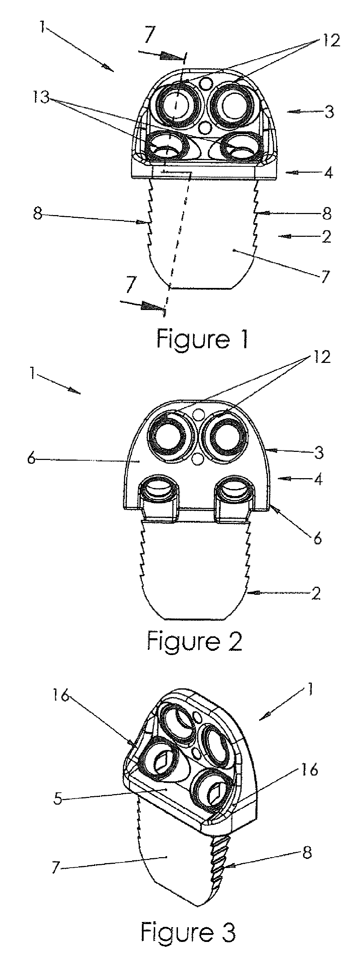

Selon l'exemple illustré aux figures 1 à 3, les parties distale 3 et intermédiaire 4 présentent chacune deux trous pour le passage des vis, lesdits trous peuvent présenter une forme conique. La grande ouverture des trous accessible du côté interne de la plaque constitue l'entrée de ceux-ci, tandis que la petite ouverture desdits trous débouche sur le côté externe de la plaque destiné à être appliquée contre la corticale de l'extrémité distale du calcanéum pour ce,qui est de la face distale de la plaque, et contre la partie spongieuse de l'extrémité proximale du calcanéum pour la concavité de la partie intermédiaire.

La plaque de translation calcanéenne 1 selon l'invention est constituée d'un matériau biocompatible, rigide mais expansible pour permettre le verrouillage des têtes de vis tout en assurant une rigidité suffisante pour fixer entre elles les deux parties de l'os issues de l'ostéotomie.

Par exemple, la plaque 1 est en polyétheréthercétone (PEEK), par exemple du type commercialisé sous la marque déposée « PEEK-OPTIMA ».

Selon un mode de réalisation avantageux, les parois latérales des passages des trous 12 et 13 destinées à recevoir les têtes de vis orthopédiques 14 et 15 sont expansibles en direction radiale de manière à permettre la compression desdites têtes de vis qui y sont insérées, assurant ainsi le verrouillage ou blocage des vis dans la plaque de translation calcanéenne et supprimant toute possibilité de recul après vissage dans la matière osseuse, ce qui pérennise la translation résultant de l'opération d'ostéotomie.

Les vis 15 se verrouillant dans les trous 13 de la partie intermédiaire 4 ont déjà un angle de divergence accroissant la tenue du dispositif et le maintien dans plusieurs plans. Le verrouillage permet aux vis 14 et 15 d'être solidaires de la plaque.

Selon un mode d'exécution, les vis 15 qui se logent dans l'extrémité proximale du calcanéum sont déjà angulées entre 10° et 50°. Selon un mode de réalisation avantageux, au moins l'une des vis utilisées avec la plaque selon l'invention possède une tête filetée.

Avantageusement, le matériau biocompatible constituant l'implant est chargé en fibres carbone à hauteur de 30%, ce qui permet son retrait futur en cas de besoin, tout en assurant la rigidité nécessaire.

Selon un mode de réalisation, le verrouillage des têtes de vis dans le PEEK (compression) est inspiré du système breveté (FR-2.845.588 « Dispositif d'ostéosynthèse autobloquant », au nom de Biotech international) et désigné par la marque déposée « EASYLOCK ».

Selon un exemple caractéristique, la partie intermédiaire 4 de la plaque 1 selon l'invention présente des goussets de renfort 16 sur ses bords latéraux.

Selon un mode de réalisation avantageux de l'invention, les parties de la plaque qui ne sont pas en contact avec l'os, c'est-à-dire la partie intermédiaire 4 présentant un profil en forme d'équerre, la face de la partie distale de la plaque qui n'est pas en appuie contre l'os ainsi que les éventuels goussets 16, présentent des bords arrondis de manière à augmenter la surface de contact et diminuer l'angle d'attaque afin d'éviter les lésions occasionnées par le coefficient de friction entre la peau et la plaque de translation.

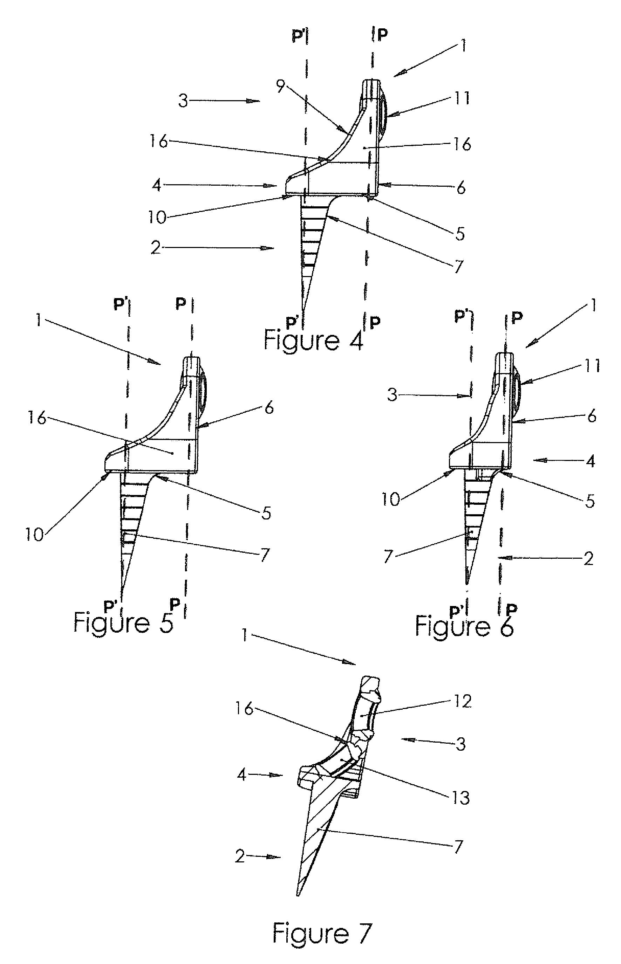

Pour permettre de s'adapter au besoin spécifique du patient, chaque cas étant différent, les implants selon l'invention seront mis à la disposition des chirurgiens sous forme de gammes comprenant, chacune, une pluralité de plaques de translation calcanéenne de dimensions différentes (par exemples figures 4, 5 et 6).

La plaque de translation calcanéenne selon l'invention procure plusieurs avantages intéressants et notamment une :
- très grande stabilité ;
- absence d'encombrement des têtes de vis en dehors de la plaque ;
- conformité anatomique de la plaque qui s'adapte à la forme de l'os ;
- très bonne résistance au poids de la marche.

## Revendications

1. Plaque de translation du calcanéum (1) comprenant deux parties extrêmes, soit une partie proximale d'ancrage (2) et une partie distale de fixation (3), orientées dans des directions opposées, lesdites parties proximale et distale se raccordant à une partie intermédiaire (4), **caractérisée en ce que** lesdites parties proximale et distale sont disposées dans des plans parallèles espacés (P-P, P'-P'), et **en ce que** la partie intermédiaire présente un profil général en équerre et comportant deux surfaces d'appui perpendiculaires, la partie proximale d'ancrage s'étendant à partir de l'une (5) de ces surfaces et perpendiculairement à celle-ci, tandis que la partie distale de fixation s'étend dans le prolongement de l'autre surface d'appui (6).

2. Plaque de translation calcanéenne selon la revendication 1, **caractérisée en ce que** la partie proximale d'ancrage (2) est constituée par une lame (7) présentant un profil s'amincissant en direction de son extrémité libre.

3. Plaque de translation calcanéenne suivant la revendication 2, **caractérisée en ce que** ladite lame (7) possède des crans anti-retour (8).

4. Plaque de translation calcanéenne selon la revendication 3, **caractérisée en ce que** lesdits crans anti-retour (8) sont disposés sur les bords latéraux de la partie proximale d'ancrage (2).

5. Plaque de translation calcanéenne suivant l'une quelconque des revendications 1 à 4, **caractérisée en ce que** la partie intermédiaire (4) présente un épaulement (10) au niveau de sa surface d'appui reliée à la partie proximale d'ancrage (2).

6. Plaque de translation calcanéenne selon l'une quelconque des revendications 1 à 5, **caractérisée en ce que** la partie distale de fixation (3) comporte au moins un trou (12) pour le passage d'au moins une vis de fixation (14).

7. Plaque de translation calcanéenne selon l'une quelconque des revendications 1 à 6, **caractérisée en ce que** la partie intermédiaire (4) présente une concavité (9) comportant un ou plusieurs trous (13) pour le passage d'une ou plusieurs vis de fixation (15).

8. Plaque de translation calcanéenne selon l'une quelconque des revendications 1 à 7, **caractérisée en ce que** les parties distale (3) et intermédiaire (4), sont toutes deux munies de trous (12 et 13) pour le passage des vis (14 et 15), l'axe des trous (13) de la partie intermédiaire (4) étant incliné par rapport à l'axe des trous (12) de la partie distale (3).

9. Plaque de translation calcanéenne selon la revendication 8, **caractérisée en ce que** les parties distale (3) et intermédiaire (4) présentent, chacune, deux trous (12 ou 13) pour le passage des vis (14 ou 15).

10. Plaque de translation calcanéenne selon l'une quelconque des revendications 6 à 9, **caractérisée en ce que** lesdits trous (12 et 13) pour le passage des vis orthopédiques ont une forme conique.

11. Plaque de translation calcanéenne suivant l'une quelconque des revendications 1 à 10, **caractérisée en ce que** la partie distale de la plaque présente un bosselage (11) sur sa face destinée à venir au contact de l'os.

12. Plaque de translation calcanéenne suivant l'une quelconque des revendications 1 à 11, **caractérisée en ce que** la partie intermédiaire (4) de ladite plaque (1) présente, sur ses bords latéraux, des goussets de renfort (16).

## Patentansprüche

1. Fersenbeintranslationsplatte (1) mit zwei Endabschnitten, einen proximalen Verankerungsteil (2) und einen distalen Befestigungsteil (3), die in entgegengesetzten Richtungen ausgerichtet sind, wobei die proximalen und distalen Abschnitte mit einem Zwischenabschnitt (4) verbunden sind, **dadurch gekennzeichnet, dass** die proximalen und distalen Abschnitte in beabstandeten parallelen Ebenen (PP; P-P') angeordnet sind und dass der Zwischenabschnitt ein grundsätzlich quadratisches Profil und zwei senkrechte Lagerflächen aufweist, wobei sich der proximale Verankerungsteil von einer (5) dieser Flächen erstreckt, und senkrecht dazu, während sich der distale Befestigungsabschnitt in der Verlängerung der anderen Anlagefläche (6) erstreckt.

2. Fersenbeintranslationsplatte (1) nach Anspruch 1 **dadurch gekennzeichnet, dass** der proximale Verankerungsabschnitt (2) durch eine Schneide (7) gebildet wird, das ein Profil darstellt, welches sich zu seinem freien Ende hin verjüngt.

3. Fersenbeintranslationsplatte nach Anspruch 2, **dadurch gekennzeichnet, dass** die Klinge Widerhaken (8) aufweist.

4. Fersenbeintranslationsplatte als 3. **dadurch gekennzeichnet, dass** die genannten Widerhaken (8) an den Seitenkanten des proximalen Verankerungsteils (2) angeordnet sind.

5. Fersenbeintranslationsplatte nach einem der Ansprüche 1 bis 4, **dadurch gekennzeichnet, dass** der Zwischenabschnitt (4) eine Schulter (10) an seiner Anlagefläche an dem proximalen Ankerabschnitt (2) aufweist.

6. Fersenbeintranslationsplatte nach einem der Ansprüche 1 bis 5, **dadurch gekennzeichnet, dass** der distale Befestigungsteil (3) mindestens ein Loch (12) für den Durchgang von mindestens einer Befestigungsschraube aufweist (14).

7. Fersenbeintranslationsplatte nach einem der Ansprüche 1 bis 6, **dadurch gekennzeichnet, dass** der Zwischenabschnitt (4) eine Konkavität (9) mit einem oder mehreren Löchern (13) für den Durchgang einer oder mehrerer Befestigungsschrauben (15) aufweist.

8. Fersenbeintranslationsplatte nach einem der Ansprüche 1 bis 7, **dadurch gekennzeichnet, dass** die distalen Abschnitte (3) und der Zwischenabschnitt (4) beide mit Löchern (12 und 13) für den Durchtritt von Schrauben (14 und 15), die Achse der Löcher (13) des Zwischenabschnitts (4) relativ zur Achse der Löcher (12) des distalen Abschnitts (3) geneigt sind.

9. Fersenbeintranslationsplatte Platte nach Anspruch 8, **dadurch gekennzeichnet, dass** die distalen Abschnitte (3) und der Zwischenabschnitt (4) jeweils zwei Bohrungen (12 bzw. 13) für den Durchtritt der Schrauben (14 oder 15) aufweisen.

10. Fersenbeintranslationsplatte nach einem der Ansprüche 6 bis 9, **dadurch gekennzeichnet, dass** die Löcher (12 und 13) für den Durchtritt von orthopädischen Schrauben eine konische Form aufweisen.

11. Fersenbeintranslationsplatte nach einem der Ansprüche 1 bis 10. **dadurch gekennzeichnet, dass** der distale Abschnitt der Platte eine Prägung (11) auf seiner Fläche aufweist, der in Berührung mit dem Knochen kommen soll.

12. Fersenbeintranslationsplatte nach einem der Ansprüche 1 bis 11, **dadurch gekennzeichnet, dass** der Zwischenabschnitt (4) der Platte (1), an seinen seitlichen Rändern Verstärkungszwickel (16) aufweist.

## Claims

1. Calcaneum translation plate (1) comprising two end portions, i.e. a proximal anchoring portion (2) and a distal fixing portion (3), oriented in opposing directions, said proximal and distal portions connecting to an intermediate portion (4), **characterised in that** said proximal and distal portions are arranged in spaced parallel planes (P-P, P'-P') and **in that** the intermediate portion has a square general profile, and comprising two perpendicular bearing surfaces, the proximal anchoring portion extending from one (5) of said surfaces and perpendicular thereto, whereas the distal fixing portion extends in the extension of the other bearing surface (6).

2. Calcaneum translation plate according to claim 1, **characterised in that** the proximal anchoring portion (2) is constituted by a blade (7) having a profile tapering towards its free end.

3. Calcaneum translation plate according to claim 2, **characterised in that** said blade (7) has anti-retum notches (8).

4. Calcaneum translation plate according to claim 3, **characterised in that** said anti-retum notches (8) are arranged on the lateral edges of the proximal anchoring portion (2).

5. Calcaneum translation plate according to any one of claims 1 to 4, **characterised in that** the intermediate portion (4) has a shoulder (10) at the level of its bearing surface connected to the proximal anchoring portion (2).

6. Calcaneum translation plate according to any one of claims 1 to 5, **characterised in that** the distal fixing portion (3) has at least one hole (12) for the passage of at least one fixing screw (14).

7. Calcaneum translation plate according to any one of claims 1 to 6, **characterised in that** the intermediate portion (4) has a concavity (9) having one or more holes (13) for the passage of one or more fixing screws (15).

8. Calcaneum translation plate according to any one of claims 1 to 7, **characterised in that** the distal (3) portion and the intermediate portion (4) are both provided with holes (12 and 13) for the passage of the screws (14 and 15), the axis of the holes (13) of the intermediate portion (4) being inclined relative to the axis of the holes (12) of the distal portion (3)

9. Calcaneum translation plate according to claim 8, **characterised in that** the distal (3) portion and the intermediate portion (4) each have two holes (12 or 13) for the passage of the screws (14 or 15).

10. Calcaneum translation plate according to any one of claims 6 to 9, **characterised in that** said holes (12 and 13) for the passage of orthopaedic screws have a conical shape.

11. Calcaneum translation plate according to any one of claims 1 to 10, **characterised in that** the distal portion of the plate has an embossment (11) on its face intended to come into contact with the bone.

12. Calcaneum translation plate according to any one of claims 1 to 11, **characterised in that** the intermediate portion (4) of said plate (1) has, on its lateral edges, reinforcing gussets (16).
